(19) Europäisches Patentamt
European Patent Office
Office européen des brevets

(11) Veröffentlichungsnummer: **0 098 567**
**A2**

(12) # EUROPÄISCHE PATENTANMELDUNG

(21) Anmeldenummer: **83106511.5**

(22) Anmeldetag: **04.07.83**

(51) Int. Cl.³: **C 07 F 9/38**, C 07 F 9/40, A 61 K 31/66

(30) Priorität: **05.07.82 DE 3225469**

(43) Veröffentlichungstag der Anmeldung: **18.01.84**
**Patentblatt 84/3**

(84) Benannte Vertragsstaaten: **AT BE CH DE FR GB IT LI LU NL SE**

(71) Anmelder: **SCHERING AKTIENGESELLSCHAFT Berlin und Bergkamen,**
**Müllerstrasse 170/178 Postfach 65 03 11,**
**D-1000 Berlin 65 (DE)**

(72) Erfinder: **Biere, Helmut, Dr., Zeltinger Strasse 15,**
**D-1000 Berlin 28 (DE)**
Erfinder: **Rufer, Clemens, Dr., Westhofener Weg 14,**
**D-1000 Berlin 38 (DE)**
Erfinder: **Böttcher, Irmgard, Dr., Frobenstrasse 46,**
**CH-4000 Basel (CH)**

(54) **Diphosphonsäure-Derivate und diese enthaltende pharmazeutische Präparate.**

(57) Diphosphonsäure-Derivate der allgemeinen Formel I

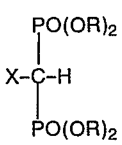

(I),

worin
X eine Cyanogruppe, eine 2-Cyanoethylgruppe, eine Dimethylaminomethylengruppe oder eine 3-Aminopropylgruppe und R ein Wasserstoffatom, ein Alkalimetallatom oder eine 1 bis 4 Kohlenstoffatome enthaltende Alkylgruppe bedeuten, mit Ausnahme des Cyanomethan-bis(phosphonsäure-diethyl-esters), sind pharmakologisch wirksame Substanzen.

EP 0 098 567 A2

Die Erfindung betrifft die in den Patentansprüchen gekennzeichneten Diphosphonsäure-Derivate, ein Verfahren zu ihrer Herstellung und pharmazeutische Präparate, die diese Verbindungen enthalten.

Die erfindungsgemäßen Verbindungen haben überraschenderweise eine ausgeprägte antiinflammatorische und antiarthritische Wirksamkeit. Darüberhinaus zeichnen sie sich dadurch aus, daß sie unter anderem imstande sind, die Aufbau- und Abbau-Leistung der Knochenzellen (Osteoblasten/Osteoklasten) so zu beeinflussen, daß kurative Effekte bei Ratten mit induzierter Arthritis eindeutig nachweisbar sind.

Mit dieser antiarthritischen Wirksamkeit der erfindungsgemäßen Verbindungen ist die Grundlage für eine Therapie der Rheumatoiden Arthritis, der Osteoarthritis, Spondylitis ankylosans und anderer verwandter Erkrankungen, besonders des Kollagens und des Skelettsystems (Osteoporose, Pagetskrankheit) geschaffen. Darüber hinaus können die Phosphonate als gute Komplexbildner für Calcium überall dort therapeutisch sinnvoll eingesetzt werden, wo ein gestörter Ca-Stoffwechsel als Ursache für eine Erkrankung erkannt wurde, z.B. bei cardiovaskulären Erkrankungen, ektopischen Calcifikationen etc..

Die Verbindungen können in Form ihrer Ester, Halbester - vorzugsweise jedoch in Form der freien Phosphonsäuren bzw. deren physiologisch verträglichen Salzen mit Alkali-, Erdalkalihydroxiden oder verträglichen organischen Basen - angewendet werden. Als galenische Formulierungen sind Kapseln, Tabletten, Dragees, Suppositorien, aber auch Injektionslösungen und dermale Zubereitungen geeignet. Auch eine lokale Anwendung zur Behandlung dermaler oder systemischer Erkrankungen ist möglich.

Die Herstellung der Diphosphonsäure-Derivate erfolgt nach Methoden wie sie dem Fachmann wohlbekannt sind und welche in den nachfolgenden Schemata dargestellt sind.

Darstellungsverfahren: Schema 1

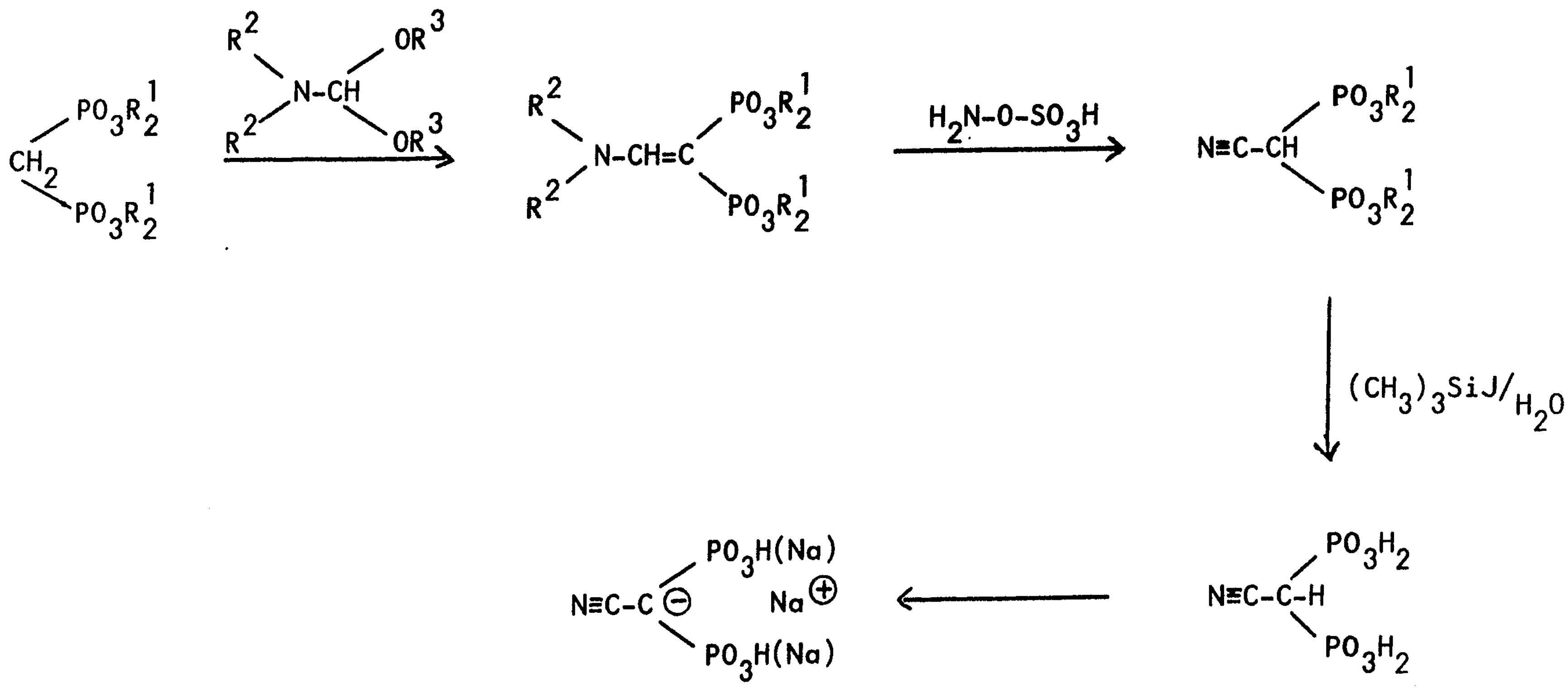

Schema 2

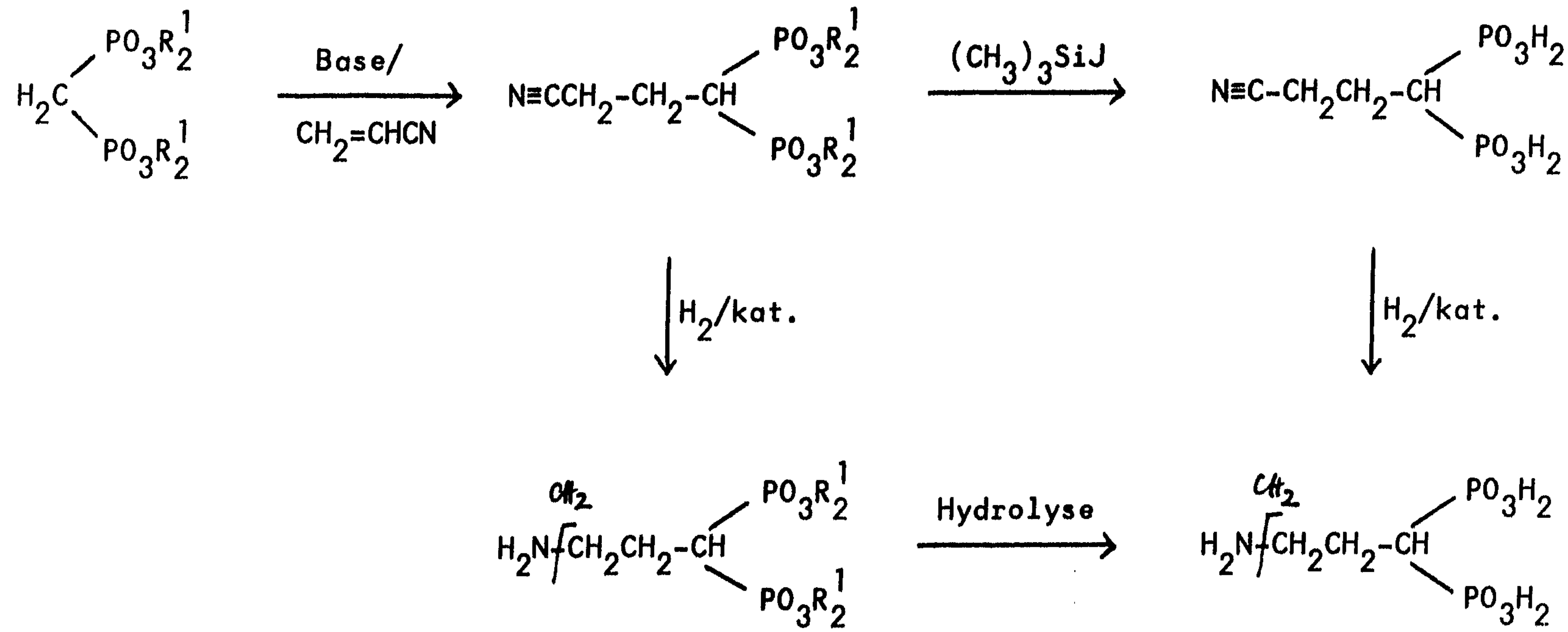

Die Umsetzung der Dimethylformamiddialkylacetale der allgemeinen Formel II mit den Methan-bis(phosphonsäuredialkylestern) der allgemeinen Formel III zu den entsprechenden Enaminen kann in an sich bekannter Weise durchgeführt werden (z.B. Enamines, G. Cook, Marcel-Dekker-Verlag New York und London, 1969, Chem. Ber. 101, 1968, 4048,), so zum Beispiel,indem man die Komponenten in Gegenwart von Basen (Kalium-tert.-butylat etc) erhitzt.

Die sich gewünschtenfalls anschließende Umsetzung der Enamine erfolgt ebenfalls unter dem Fachmann wohlbekannten Bedingungen ( Europäische Patentanmeldung 16 978), indem man beispielsweise diese Verbindungen in wässriger Lösung bei einem $p_H$-Wert von 0 bis 1 mit der Hydroxylamin-O-sulfonsäure umsetzt.

Die sich gegebenenfalls anschließende Verseifung der Ester kann mittels Mineralsäuren ( Z.B. halbkonzentrierte Salzsäure oder Schwefelsäure) durchgeführt werden. Besonders schonend gelingt die Spaltung in einem inerten Lösungsmittel ( z.B: Diethylether, Diisopropylether, Dioxan, Tetrahydrofuran, Dichlormethan, Chloroform oder Tetrachlorkohlenstoff) mit Trimethylsilyljodid. Zur Salzbildung werden die freien Säuren in üblicher Weise mit den entsprechenden Basen umgesetzt.

Die Kondensation der Methan-bis(phosphonsäuredialkylester) der allgemeinen Formel IV mit Acrylnitril erfolgt unter den Bedingungen, die man üblicherweise zur Cyanethylierung aktiver Methylenverbindungen verwendet. So kann man beispielsweise Verbindungen der Formel IV in einem inerten Lösungsmittel ( Diethylether, Diisopropylether, Dioxan, Tetrahydrofuran etc ) mit Natrium, Kalium oder deren Hydriden in ihre Salze überführen und diese mit Acrylnitril umsetzen.

Die sich gegebenenfalls anschließende Reduktion der Nitrile zu den entsprechenden Aminen erfolgt ebenfalls unter den Bedingungen, wie sie dem Fachmann wohlbekannt sind. So kann man die Nitrile beispielsweise mit Wasserstoff in Gegenwart von Nickel- oder Platikatalysatoren hydrieren, oder mit komplexen Metallhydriden wie zum Beispiel Lithiumaluminiumhydrid reduzieren.

Die nachfolgenden Aufführungsbeispiele dienen zur Erläuterung des erfindungsgemäßen Verfahrens.

Beispiel 1

2-Dimethylamino-ethen-1,1-bis(phosphonsäure-diethylester)

Eine Mischung von 2,9 g (20 mmol) Dimethylformamid-diethylacetal und 2,9 g (10 mmol) Methan-bis(phosphonsäure-diethylester) werden mit 110 mg Kalium-tert.-butylat versetzt und unter Stickstoff 1 Stunde auf 120° C (Badtemperatur) erhitzt. Danach wird das entstandene Ethanol sowie das überschüssige Acetal im Vakuum abdestilliert. Es bleibt ein helles Öl zurück: Ausbeute 3,4 g.

Beispiel 2

Cyanomethan-bis(phosphonsäure-diethylester)

Eine Lösung von 45,2 g (0,4 mol) Hydroxylamin-O-sulfonsäure in 400 ml Wasser wird mit 68,6 g (0,2 mol) 2-Dimethylaminoethen-1,1-bis(phosphonsäure-diethylester) versetzt und 30 Minuten bei Raumtemperatur gerührt, anschließend rasch mit 3 x 100 ml Dichlormethan extrahiert. (Die organischen Extrakte werden verworfen). Die wässrige Phase wird mit 100 ml Dichlormethan versetzt und über Nacht bei Raumtemperatur weitergerührt. Nach Abtrennen der organischen Phase und Einengen erhält man 37 g.

Die wässrige Phase wird erneut mit 100 ml Dichlormethan über Nacht gerührt, wie oben beschrieben aufbereitet und man erhält 19 g.

Eine dritte gleichartige Behandlung führt zu 9 g Produkt. Insgesamt erhält man also 65 g.

Nach Kugelrohrdestillation bei 170-175° C und 0,01 mm Druck werden 49,4 g erhalten.

Beispiel 3

Cyano-methan-diphosphonsäure, Tri-Natriumsalz

Eine Lösung von 6,3 g (20 mmol) Cyanomethan-bis(phosphonsäure-diethylester) in 30 ml Tetrachlorkohlenstoff werden unter Stickstoffatmosphäre bei 0° C tropfenweise mit 12 ml Jodtrimethylsilan versetzt und anschließend noch 1 Stunde bei Raumtemperatur gerührt. Nach dem Einengen wird der Rückstand mit Aceton und Wasser versetzt und erneut im Vakuum konzentriert. Durch Extraktion mit Dichlormethan werden jodhaltige Verunreinigungen abgetrennt, dann das in Wasser lösliche Material mit 5 g Natriumhydrogencarbonat versetzt und schließlich das entstandene Tri-Natriumsalz durch Zusatz von Ethanol aus der wässrigen Lösung gefällt. Ausbeute 3,74 g. Schmelzpunkt oberhalb 300° C.

Die freie Cyanomethan-1,1-diphosphonsäure kann als Öl erhalten werden durch Behandlung des Natriumsalzes mit dem Ionenaustauscher Amberlite IR 120.

Beispiel 4

3-Cyanopropan-1,1-bis(phosphonsäure-diethylester)

Eine Suspension von 2,4 g Natriumhydrid (80 %) in 100 ml Dimethoxyethan werden bei 0° C tropfenweise mit einer Lösung von 23,1 g Methan-bis(phosphonsäure-diethylester) in 20 ml Dimethoxyethan versetzt. Nach beendeter Gasentwicklung wird noch 15 Minuten bei 0° C nachgerührt, dann mit 4,25 g Acrylnitril versetzt und 5 Stunden auf 80° C erhitzt. Nach Stehen über Nacht wird mit 5 ml Eisessig versetzt, dann das Dimethoxyethan im Vakuum destilliert und der Rückstand an Kieselgel (Toluol-Aceton) chromatographiert. Die Nitrilfraktion wird anschließend noch einmal am Kugelrohr destilliert (Siedepunkt 180-190° C, 0,03 mm). Ausbeute 12 g.

Beispiel 5

3-Cyanopropan-1,1-bis(phosphonsäure), Di-Natriumsalz

Analog Beispiel 3 wird aus 3-Cyanopropan-1,1-bis(phosphonsäure-diethylester) [Beispiel 4] durch Spaltung mit Jodtrimethylsilan die 3-Cyanopropan-1,1-diphosphonsäure dargestellt und in das Di-Natriumsalz überführt. Ausbeute 71 %. Schmelzpunkt oberhalb 300° C.

Beispiel 6

4-Aminobutan-1,1-bis(phosphonsäure-diethylester)

Eine Lösung von 2,44 g (7,1 mmol) 3-Cyanopropan-1,1-bis (phosphonsäure-diethylester) in 40 ml ethanolischer Salzsäure (0,7 N) wird mit 285 mg Platindioxid versetzt und bei Raumtemperatur hydriert. Nach Aufnahme der theoretischen Menge Wasserstoff wird vom Katalysator abgesaugt, dieser mit Ethanol nachgewaschen, und die vereinigte Ethanol-Phase im Vakuum eingeengt. Der Rückstand wird mit wenig Wasser und Natriumhydrogencarbonat behandelt und erschöpfend mit Dichlormethan extrahiert. Der Rückstand der Dichlormethanphase beträgt 1,9 g.

Beispiel 7

4-Aminobutan-1,1-diphosphonsäure, Di-Natriumsalz

Die Darstellung wird aus dem Natriumsalz des Beispiels 5 durch katalytische Hydrierung mit Platindioxid in wässriger Lösung vorgenommen. Ausbeute 80 %; Schmelzpunkt oberhalb 300° C (aus Dimethylformamid).

## Patentansprüche

1. Diphosphonsäure-Derivate der allgemeinen Formel I

$$\begin{array}{l} \quad PO(OR)_2 \\ \quad\, | \\ X-C-H \\ \quad\, | \\ \quad PO(OR)_2 \end{array} \qquad (I),$$

worin
X eine Cyanogruppe, eine 2-Cyanoethylgruppe, eine Dimethylaminomethylengruppe oder eine 3-Aminopropylgruppe und
R ein Wasserstoffatom, ein Alkalimetallatom oder eine 1 bis 4 Kohlenstoffatome enthaltende Alkylgruppe bedeuten, mit Ausnahme des Cyanomethan-bis(phosphonsäure-diethylesters).

2. 2-Dimethylamino-ethen-1,1-bis(phosphonsäure-diethylester.

3. Cyano-methan-diphosphonsäure.

4. Trinatriumsalz der Cyano-methan-diphosphonsäure.

5. 3-Cyanopropan-1,1-bis(phosphonsäurediethylester).

6. 3-Cyanopropan-1,1-bis(phosphonsäure).

7. Dinatriumsalz der 3-Cyanopropan-1,1-bis(phosphonsäure).

8. 4-Aminobutan-1,1-bis(phosphonsäure-diethylester).

9. 4-Aminobutan-1,1-bis-diphosphonsäure.

10. Dinatriumsalz der 4-Aminobutan-1,1-bis-diphosphonsäure.

11. Pharmazeutische Präparate gekennzeichnet durch einen Gehalt an einem Diphosphonsäure-Derivat gemäß Anspruch 1 bis 10.

12. Verfahren zur Herstellung von Diphosphonsäure-Derivaten der allgemeinen Formel I

$$\begin{array}{c} PO(OR)_2 \\ | \\ X-C-H \\ | \\ PO(OR)_2 \end{array} \qquad (I),$$

worin
X eine Cyanogruppe, oder eine Dimethylaminomethylengruppe und
R ein Wasserstoffatom, ein Alkalimetallatom oder eine 1 bis 4 Kohlenstoffatome enthaltende Alkylgruppe bedeuten, mit Ausnahme des Cyanomethan-bis(phosphonsäure-diethylesters),
dadurch gekennzeichnet, daß man in ansich bekannter Weise ein Dimethylformamiddialkylacetal der allgemeinen Formel II

$$(CH_3)_2NCH\ (OR')_2 \qquad (II),$$

worin R' eine 1 bis 4 Kohlenstoffatome enthaltende Alkylgruppe bedeutet,
mit einem Methan-bis(phosphonsäuredialkylester) der allgemeinen Formel III

$$CH_2(PO_3R_2)_2 \qquad (III),$$

worin R die obengenannte Bedeutung besitzt, umsetzt und gewünschtenfalls das erhaltene Enamin mit Hydroxylamin-O-sulfonsäure behandelt, vorhandene Estergruppen verseift und die freien Säuren in ihre Salze überführt.

13. Verfahren zur Herstellung von Diphosphonsäure-Derivaten der allgemeinen Formel I

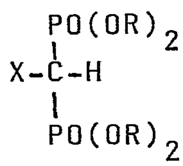

(I),

worin

X eine 2-Cyanoethylgruppe oder eine 3-Aminopropylgruppe und

R ein Wasserstoffatom, ein Alkalimetallatom oder eine 1 bis 4 Kohlenstoffatome enthaltende Alkylgruppe bedeuten, dadurch gekennzeichnet, daß man in an sich bekannter Weise einen Methan-bis(phosphonsäuredialkylester) der allgemeinen Formel IV

$$CH_2(PO_3R_2)_2 \qquad (IV),$$

worin R die obengenannte Bedeutung besitzt, mit Acrylnitril umsetzt und gewünschtenfalls in beliebiger Reihenfolge, die erhaltenen Nitrile zu den entsprechenden Aminen reduziert und/oder die erhaltenen Ester verseift und die freien Säuren in ihre Salze überführt.